Europäisches Patentamt

European Patent Office

Office européen des brevets

(11) Publication number: 0 345 559
A2

# (12) EUROPEAN PATENT APPLICATION

(21) Application number: 89109500.2

(22) Date of filing: 26.05.89

(51) Int. Cl.4: **C12N 7/00 , A61K 39/21 , G01N 33/569**

(30) Priority: 10.06.88 US 205200

(43) Date of publication of application:
13.12.89 Bulletin 89/50

(84) Designated Contracting States:
AT BE CH DE ES FR GB IT LI NL

(71) Applicant: **ABBOTT LABORATORIES**
**One Abbott Park Road**
**Abbott Park, IL 60064-3500(US)**

(72) Inventor: **Barin, Francis R.**
**25 Rue Terre Pierre Valence**
**F-37100 Tours(FR)**

(74) Representative: **Modiano, Guido et al**
**MODIANO, JOSIF, PISANTY & STAUB**
**Modiano & Associati Via Meravigli, 16**
**I-20123 Milano(IT)**

(54) HIV-2 variants.

(57) Disclosed are novel HIV-2 strains characterized by the presence of gp 150 antigens and useful as reference strains in the detection of, e.g., anti-HIV-2 antibodies.

FIG. 2

EP 0 345 559 A2

## HIV-2 VARIANTS

BACKGROUND OF THE INVENTION

Field of the Invention

The present invention relates generally to novel strains of HIV-2 retrovirus and more particularly to "TY type" strains of HIV-2 retrovirus as exemplified by the HIV-2 TY strain deposited with the European Collection Of Animal Cell Cultures (ECACC), Porton Down, Salisbury, Wiltshire, United Kingdom.

Description of Related Art

It has been determined that two related but distinct viruses can cause Acquired Immune Deficiency Syndrome (AIDS): Human Immunodeficiency Virus One (HIV-1) and Human Immunodeficiency Virus Two (HIV-2). Although different HIV-1 strains have been isolated, they share common antigenic sites on their principle proteins. This relationship permits a prototype strain to be used as a source of antigens for detection of anti-HIV-1 antibodies in the blood of infected individuals regardless of the specific infecting strain.

The first evidence of an HIV-2 type infection was discovered in West Africa in 1985 (Barin, et al., Lancet 1985; II: 1387-1389). This virus was isolated and designated HTLV-IV (Kanki, et al., Science 232, 238-243 (1986)). Montagnier and his coworkers then isolated another HIV-2 type infection from West African AIDS patients in 1985 and labeled it LAV-2 (PCT WO 87/04459). Then in 1987, Albert, et al., in Aids Research and Human Retroviruses, 1987, 3: 3-10, disclosed that another HIV-2 human retrovirus from West Africa had been isolated - SBL-6669.

Evidence in the literature now indicates that the HTLV-IV (HIV-2 PK 289) isolate was probably derived from simian immunodeficiency virus MAC-251 infected cell cultures. LAV-2 and SBL-6669, however, have been associated with human immunodeficiency of a type distinguishable from HIV-1. The genomes of HIV-1 and HIV-2 are only about 50% homologous at the nucleotide level and HIV-1 prototype strains cannot be used as the source of antigens for detection of HIV-2 antibodies. As was the case with HIV-1, multiple strains of HIV-2 have been detected, making it important to determine a prototype strain for use as a source of antigens for detection of anti-HIV-2 antibodies regardless of the specific infecting strain.

Albert, et al., in their disclosure of the newly isolated SBL-6669 strain, compared this strain to HTLV-4, LAV-2 and HTLV-IIIB (Aids Research and Human Retroviruses, 1987, 3: 3-10). They found that LAV-2 and SBL-6669 readily infected and replicated in the human T-cell lines HUT78, CEM, Jurkat and U937. They also found that HTLV-IV, LAV-2, SBL-6669 and HTLV-IIIB all had an external glycoprotein with an approximate molecular size of 120kD. They also indicated that LAV-2, HTLV-IV and SBL-6669 were fully cross-reactive as all of them gave identical reactions in cross-serological analysis. Albert concluded that antigens from any of the West African isolates (HTLV-IV, LAV-II, and SBL-6669) could serve as a prototype strain to detect antibodies directed against the other two, but he cautioned that further studies would be required to determine if this would be true for other virus isolates of this variety.

BRIEF SUMMARY

The present invention provides novel "TY type" strains of HIV-2 retrovirus suitable for use as a reference or prototypic HIV-2 strain and characterized by an antigenic profile including a 150 kD glycoprotein upon analysis of viral proteins by SDS PAGE. Illustrative of the invention is the TY strain of HIV-2 retrovirus as deposited on June 10, 1988 with the ECACC, Vaccine Research and Production Laboratory, Public Health Laboratory Service, Centre for Applied Microbiology and Research, Porton Down, Salisbury, Wiltshire SP4 OJG, United Kingdom, under accession number V-88061001.

Novel strains of HIV-2 retrovirus of the invention may be characterized by an antigenic profile additionally including 110 kD and 39 kD Env gene products, 68 kD and 34 kD Pol gene products, and 25/26 kD and 17 kD Gag gene products as revealed by SDS PAGE analysis of the deposited strain.

Strains of HIV-2 retrovirus according to the invention are expected to share with the TY strain the ability to replicate in HUT 78 cells, SUP T-1 cells, and U937 cells, and the inability to replicate in CEM cells after 24 days of incubation.

Further provided by the invention are novel compositions and antigenic preparations comprising purified and isolated TY type HIV-2 retrovirus proteins characterized by the presence of a 150 kD glycoprotein such as that revealed upon analysis of TY strain viral proteins by SDS PAGE.

In another of its aspects, the invention provides methods for the in vitro detection of the presence of anti-HIV-2 antibodies in biological fluids comprising contacting a biological fluid sample with the purified antigenic preparation or the crude viral lysate of TY type strain HIV-2 proteins and detecting immunological complexes formed between antibodies in the sample and the antigens.

Further aspects and advantages of the invention will be apparent to those skilled in the art upon review of the following detailed description taken in conjunction with the Figures and the appended claims.

## BRIEF DESCRIPTION OF THE FIGURES

Figure 1 is a photograph of a Western blot in which the antigenic profiles of HIV-1 (HTLV-IIIB), HIV-2 TY and HIV-2 PK 289 as revealed by reacting with anti-HIV-1 and anti-HIV-2 serum are compared.

Figure 2 is a photograph of a Western blot in which the antigenic profiles of HIV-2 TY and HIV-2 PK 289 are compared.

## DETAILED DESCRIPTION OF THE INVENTION

The examples below illustrate the initial isolation of HIV-2 TY, the cell line in which it was propagated, the method by which its antigenic profile was determined and its potential use in diagnostic assays for the detection of infection by an HIV-2 strain.

TY strain virus was initially isolated from the plasma of a Portuguese woman (T.Y.) living in France diagnosed as having AIDS (Pneumocystic carinii pneumonia) and was used to infect a HUT 78 cell line (American Type Culture Collection, accession number TIB 161). Preliminary screening revealed that the virus strain replicated well in HUT 78, SUP T-1, and U937 cell lines, but, unlike the strains of Montagnier, et al. WO 87/04459 (deposited at ECACC under the numbers 87.01.1001 and 87.01.1002), the TY strain was not able to replicate in CEM cells after 24 days of incubation.

In order to ascertain the antigenic profile of the TY strain, 2 ml of fresh plasma were incubated with 1 X 10⁶ HUT 78 cells for 2 hours at 37°C. After washing with fresh medium, cells were cultured in RPMI 1640 (Gibco) medium supplemented with 20% fetal calf serum and antibiotics. A cytopathic effect (formation of giant cells) appeared within 2-3 weeks without dramatic cell death. After 1 month, close to 100% of the cells were HIV-2 positive by immunofluorescence. Since that time the HIV-2 TY cell line has been continuously producing HIV-2 virus and antigens.

HIV-2 TY antigens were prepared by pelleting 30 ml of supernatant fluids from a virus-infected Hut-78 culture, collected 2 days after subcultivation. The culture fluids were filtered through a 0.45 micrometer membrane, and virus was concentrated by passage through 8 ml of a 20% sucrose cushion in a SW27 rotor (Beckman) at 20,000 rpm for 2 hours. The virus pellet was then resuspended in 200 microliters of lysis buffer (0.05 mol/l "tris" HCL, pH 7.2, 0.15 mol/l NaCl, 1% 'Triton X 100', 1% sodium deoxycholate, 0.1% sodium dodecyl sulphate).

The 200 microliters of the lysed virus preparation was mixed with 200 microliters of two-fold concentrated sample buffer (4% SDS, 0.2 M dithiothreitol (DTT), 20% glycerol, and 0.0002% bromophenol blue in 0.16 M Tris-H₃PO₄, pH 6.8), and was fractionated by electrophoresis on a 12.5% polyacrylamide slab gel in the presence of sodium dodecyl sulphate (Laemmli, J.K., *Nature*, 1970; 227: 680-685). After electrophoresis, the gel was washed for 30 minutes in three changes of transfer buffer (10 mmol/l tris-HCl, pH 7.0, 2 mmol/l EDTA, 50 mmol/l NaCl).

The proteins in the gel were then passively transferred to nitrocellulose by incubating a nitrocellulose/gel "sandwich" for 36 hours at room temperature. The nitrocellulose sheet was then incubated at 37°C for 1 hour with 3% bovine serum albumin (BSA) in 10 mmol/l sodium phosphate buffer, pH 7.4, containing 0.15 mol/l NaCl (PBS), and cut into 0.5 cm strips. After three washings in PBS containing 0.20% 'Tween 20' (PBS-T), each strip was incubated overnight at room temperature in 2.5 ml of a 1:200 dilution of test serum in PBS supplemented with 1% BSA in PBS-T. The strips were washed five times with PBS-T. Affinity purified and biotin labelled sheep IgG antihuman immunoglobulins (dilution 1:200 in PBS-T; Amersham) were added to the strips and incubated at 37°C for 1 hour. After three washings with PBS-T, a 1:200 dilution of pre-formed streptavidin-biotinylated horseradish peroxidase complex (Amersham) in PBS-T was added to the strips and incubated at room temperature for 30 minutes. After three washings, peroxidase acitivity was detected colorimetrically by the addition of a fresh solution of diaminobenzidine (50 mg in 100 ml of PBS, supplemented with 0.01% hydrogen peroxide).

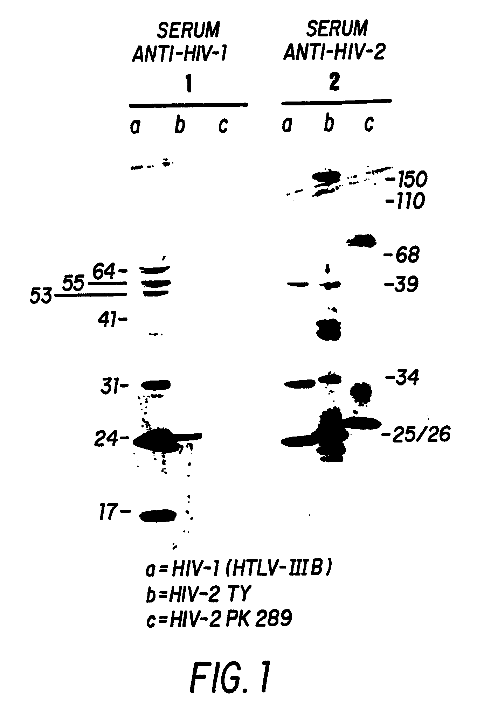

Figure 1 is a Western blot wherein the antigens in lanes a, b and c were HIV-1 (HTLV-IIIB), HIV-2 TY and HIV-2 PK 289 (obtained from Phyllis J. Kanki, Department of Cancer Biology, Harvard School of Public Health, Boston, MA), respectively, and the test sera were anti-HIV-1 in column 1 and anti-HIV-2 in column 2. As expected, neither of the HIV-2 variant proteins bound strongly to anti-HIV-1 antibodies Proteins from both strains bound strongly to anti-HIV-2 antisera and the HIV-2 TY proteins were distinguished from those of HIV-2 PK 289 by the presence of a 150 kD protein.

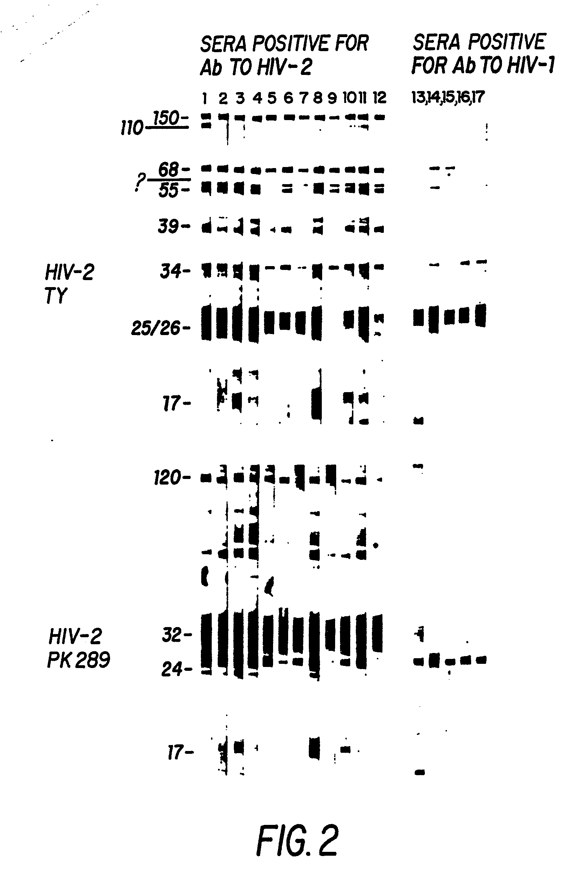

In the Figure 2 Western blots, the antigens in lanes 1-17 of the upper half are HIV-2 TY and the antigens in lanes 1-17 of the lower half are HIV-2 PK289. The test sera for lanes 1-12 in both halves of the photograph were all positive for antibodies to HIV-2 whereas the test sera for lanes 13-17 in both halves of the photograph were positive for antibodies to HIV-1. Lanes 13-17 showed very little cross-reactivity between HIV-1 antibodies and HIV-2 antigens. In lanes 1-12, HIV-2 TY antigens and HIV-2 PK 289 antigens each produced a gp 120 band. HIV-2 PK 289, howver, did not produce any gp 150 bands, whereas HIV-2 TY produced a gp 150 band with each different HIV-2 antiserum tested.

In a typical example of use as a diagnostic tool, isolated HIV-2 TY is disrupted and inactivated with detergent and sonication prior to coating polystyrene beads with the antigens. The coated beads are then incubated with a specimen diluent and human serum or plasma. Antibodies to HIV-2 in the specimen are bound to the HIV-2 TY antigens on the solid phase. After aspiration of the. unbound material and washing of the bead, goat antibody to human IgG conjugated with horseradish peroxidase (Anti-Human IgG:HRPO is incubated with the bead-antigen-antibody complex. Unbound enzyme conjugate is then aspirated and the beads washed. Next, o-Phenylenediamine (OPD) Solution containing hydrogen peroxide is added to the bead and, after incubation, a yellow-orange color develops in proportion to the amount of antibody to HIV-2 which is bound to the bead.

In another typical example of diagnostic use, polystyrene beads coated with human antibody (Ab) to HIV-2 TY are incubated with either a specimen or Control. HIV-2 antigen(s) (Ag) in the specimen bind to the bead. After incubation, unbound materials are aspirated and the beads are washed. Rabbit antibody to HIV-2 TY is then incubated with the bead and binds to the HIV-2 antigen. Unbound materials are aspirated and the beads are washed. Goat antibody to rabbit IgG conjugated with horseradish peroxidase (anti-Rabbit-IgG:HRPO) is incubated with the bead and binds to the rabbit antibody. Unbound materials are aspirated and the beads are washed. Next, o-Phenylenediamine (OPD) Solution containing hydrogen peroxide is added to the bead and, after incubation, a yellow-orange color develops in proportion to the amount of HIV-2 antigen(s) bound to the bead. The enzyme reaction is stopped by the addition of 1 N Sulfuric Acid, and the intensity of color is read using a spectrophotometer at 492 nm.

The foregoing detailed description is given for clearness of understanding only, and no unnecessary limitations should be understood therefrom, as aspects and advantages within the scope of the present invention will be apparent to those skilled in the art. For example, in its use as a diagnostic tool, HIV-2 TY antigen can be obtained by taking an infected cell line (i.e., HUT78), sonicating the cells, subjecting the solution to centrifugation so as to precipitate the cell membrane and other debris. The remaining crude viral lysate can then be applied to polystyrene beads. The coated beads are now ready to be incubated with a specimen diluent and human serum or plasma in order to detect Antibodies to HIV-2.

## Claims

1. A TY type strain of HIV-2 retrovirus characterized by an antigenic profile including a 150 kD glycoprotein upon analysis of viral proteins by SDS PAGE.

2. The strain of HIV-2 retrovirus according to claim 1 as deposited with the ECACC under accession number V-88061001.

3. The strain of HIV-2 retrovirus according to claim 1 and further characterized by an antigenic profile including 110 kD and 39 kD Env gene products, 68 kD and 34 kD Pol gene products, and 25/26 kD and 17 kD Gag gene products.

4. The strain of HIV-2 retrovirus according to claim 1 and characterized by the ability to replicate in HUT 78 cells, SUP T-1 cells, and U937 cells.

5. A composition comprising purified and isolated TY type HIV-2 retrovirus proteins.

6. A composition according to claim 5, characterized by the presence of a 150 kD glycoprotein upon analysis of viral proteins by SDS-PAGE.

7. A purified and isolated antigenic preparation comprising TY type strain of HIV-2 proteins.

8. A method for the in vitro detection of the presence of anti-HIV-2 antibodies in a biological fluid, said method comprising contacting said biological fluid with the purified antigenic preparation according to claim 7 and detecting the immunological complex formed.

9. A method for the in vitro detection of the presence of anti-HIV-2 antibodies in a biological fluid, said method comprising contacting said biological fluid with a composition according to claim 5 or 6 and detecting the immunological complex formed.

SERUM
ANTI-HIV-1

**1**

a   b   c

SERUM
ANTI-HIV-2

**2**

a   b   c

−150
−110

−68

53 —— 55 — 64−
41−

−39

31−

−34

24−

−25/26

17−

a = HIV-1 (HTLV-IIIB)
b = HIV-2 TY
c = HIV-2 PK 289

*FIG. 1*

FIG. 2